(19) Europäisches Patentamt
European Patent Office
Office européen des brevets

(11) **EP 0 943 638 B1**

(12) **FASCICULE DE BREVET EUROPEEN**

(45) Date de publication et mention
de la délivrance du brevet:
**28.11.2007 Bulletin 2007/48**

(51) Int Cl.:
***C08G 18/80*** *(2006.01)*

(21) Numéro de dépôt: **99112178.1**

(22) Date de dépôt: **26.04.1995**

(54) **Isocyanates masqués au moyen de composés hydroxyaromatiques**

Mit hydroxyaromaten blockierte Isocyanate

Isocyanates blocked with hydroxyaromatic compounds

(84) Etats contractants désignés:
**AT BE CH DE DK ES FR GB GR IE IT LI LU MC NL PT SE**

(30) Priorité: **04.05.1994 FR 9405436**

(43) Date de publication de la demande:
**22.09.1999 Bulletin 1999/38**

(62) Numéro(s) de document de la (des) demande(s) initiale(s) en application de l'article 76 CBE:
**95400934.6 / 0 680 984**

(73) Titulaire: **RHODIA CHIMIE**
**92408 Courbevoie Cédex (FR)**

(72) Inventeurs:
• **Ardaud, Pierre**
**69110 Sainte-Foy-Les-Lyon (FR)**
• **Bernard, Jean-Marie**
**69440 Mornant (FR)**

(74) Mandataire: **Jacobson, Claude et al**
**Cabinet Lavoix**
**2, Place d'Estienne d'Orves**
**75441 Paris Cedex 09 (FR)**

(56) Documents cités:
**EP-A- 0 214 495       EP-A- 0 562 394**
**FR-A- 2 266 725       FR-A- 2 370 764**
**US-A- 3 317 463**

**Description**

La présente invention a pour objet une nouvelle famille d'isocyanates masqués. Elle concerne plus particulièrement des isocyanates masqués au moyen de dérivés hydroxyaromatiques, et leur utilisation dans les techniques de revêtement au moyen de poudres.

Pour des raisons liées à la protection de l'environnement et à la sécurité du travail, on cherche à éliminer de plus en plus dans les techniques de revêtement, et notamment de peinture, l'utilisation des solvants.

Dans ce contexte, des techniques de revêtement au moyen de poudres se développent de plus en plus.

Les isocyanates masqués commencent à être utilisés mais leur emploi est limité par le peu de composés répondant aux exigences de la chimie des poudres.

Une première difficulté réside dans la difficulté à trouver des isocyanates ou mélanges d'isocyanates masqués qui restent sous forme de poudre dans les conditions de stockage usuelles, conditions qui peuvent varier beaucoup d'un endroit à un autre. Cela implique que ces composés aient un point de fusion et/ou de transition vitreuse (Tg) relativement élevé.

Les dérivés, objet de la présente étude, n'ont pas toujours un point de fusion franc. aussi dans ce cas détermine-t-on un point de fusion apparent soit au banc Koffler soit à l'aide d'une méthode de type dite capillaire (par exemple point de fusion dit "de büchi").

Un point de transition vitreuse peut être mesuré par les techniques d'analyse thermique différentielle (ATD).

Il faut également que ces composés aient des points de transition vitreuse et des points de fusion suffisamment bas pour qu'ils puissent réagir dans les conditions d'utilisation des poudres.

Il faut en outre que les composés issus des réactions de réticulation ne soient délétères ni pour la santé humaine ou animale, ni pour l'environnement.

FR 2 266 725 décrit une composition de résine polyester en poudre pour peinture pulvérulente, obtenue par mélange d'un polyester à l'état fondu avec un polyisocyanate bloqué par un p-hydroxybenzoate d'alkyle, dans lequel la fraction alkyle contient moins de 5 atomes de carbone, le polyester étant lui-même obtenu par dépolymérisation d'un polyester de haut degré de polymérisation et de viscosité intrinsèque égale ou supérieure à 0,4, par un alcool non volatil ou un ester présentant au moins un groupe hydroxyalcoolique, le polyester fondant à une température comprise entre 45 et 120°C, et présentant des groupes hydroxy terminaux ainsi qu'un degré moyen de polymérisation compris entre environ 5 et environ 50.

EP 0 562 394 décrit une préparation poudre de polyuréthane expansible contenant un agent d'expansion obtenue en faisant réagir une émulsion d'un composé comprenant des fonctions NCO libres ou réagissant avec des fonctions NCO libres avec un composé comportant 2 atomes d'hydrogène réagissant avec la fonction NCO libre ou réciproquement comportant deux groupes NCO libres, la réaction pouvant avoir lieu en présence d'un composé comportant une partie des groupes NCO bloqués.

Le composé comportant des groupes NCO en partie bloqués est le produit de réaction de l'isocyanurate du 1,6-hexaméthylène diisocyanate avec un ester d'acide hydroxybenzoïque ainsi qu'un polyol de masse moléculaire 400-60 000 ou est un polyisocyanate comprenant des groupes uretdione ou un produit de réaction de ce composé polyisocyanate comprenant des groupes uretdione et d'un ou plusieurs composés comprenant des fonctions réactives vis à vis de NCO.

Un des buts de la présente invention est de fournir une nouvelle famille d'isocyanates bloqués qui réponde aux contraintes évoquées ci-dessus.

Un autre but de la présente invention est de fournir des compositions utilisables dans le revêtement au moyen de poudres et qui contiennent des isocyanates bloqués.

Un autre but de la présente invention est de fournir un procédé de synthèse des isocyanates répondant aux contraintes ci-dessus.

Un autre but de la présente invention est de fournir un procédé d'utilisation des isocyanates masqués selon la présente invention et répondant aux contraintes ci-dessus.

Selon la présente invention, ces buts sont atteints au moyen d'isocyanates masqués, purs ou en mélange, et qui sont issus de la condensation d'un dérivé aromatique hydroxylé sur le noyau et portant une fonction choisie parmi les fonctions nitriles et de préférence carbonyles avec un isocyanate.

Il convient de choisir parmi les membres de cette famille ceux pour lesquels il est possible de déterminer un point de fusion apparent, cette mesure étant réalisée à température ambiante (20°C). Ce point de fusion doit être au moins égal à 30°C (un chiffre significatif), avantageusement à 50°C.

Il est souhaitable que les produits ne mottent pas. Donc on choisit des composés qui broyés, et stockés à température ambiante présentent une granulométrie similaire à 24 heures d'intervalle.

Le caractère mottant est en général peu ou prou lié au point de transition vitreuse (Tg). Ainsi, les composés préférés sont ceux qui présentent un point de transition vitreuse (Tg) au moins égal à 10°C (deux chiffre significatifs), avantageusement à 20°C (un chiffre significatif, de préférence deux), de préférence à 30°C (deux chiffres significatifs).

Ainsi, parmi les parahydroxybenzoates d'alcoyle, le choix des alcoyles peut être critique : les alcoyles de plus de deux carbones qui sont linéaires sont soit de point de fusion insuffisamment élevé, soit sont mellifluents en cristallisant seulement au bout d'une durée longue allant d'une semaine à plusieurs mois ce qui les rend difficiles d'usage. Ainsi le n-propyle, le n-butyle et plus généralement les n-alcoyle sont d'usage difficiles et donc ne sont pas préférés. En outre, les chaînes longues pour des raisons similaires sont aussi à éviter, notamment celles dont le nombre de carbone est supérieur à 6.

Le cas de l'éthyle est intermédiaire et donne des résultats acceptables mais non excellents. L'isopropyle et surtout le méthyle sont préférés.

Les fonctions nitriles et de préférence carbonyles peuvent être liées au noyau soit par une simple liaison, soit par l'intermédiaire d'un chaînon lequel peut être chalcogène, azote ou phosphore porteur d'un hydrogène ou d'un substituant ou méthylène éventuellement substitué.

Compte tenu du fait que l'effet électroattracteur diminue ou disparaît par l'intercalation d'un chaînon entre le groupe électroattracteur et le noyau, la liaison directe entre le noyau et le groupe électroattracteur est préférée, à moins qu'il n'y ait déjà un groupe électroattracteur sur le noyau, ou que celui-ci soit naturellement pauvre en électrons (par exemple hétérocycle à six chaînons).

Les chaînons chalcogènes ou uniquement porteurs d'hydrogène et/ou dans une moindre mesure de méthyle sont préférés. Les chaînons à base d'élément(s) de la deuxième ligne (ligne de l'oxygène) du tableau périodique des éléments sont également préférés.

Selon la présente invention, isocyanate masqué, pur ou en mélange, est issu d'un polyisocyanate, c'est-à-dire possédant au moins deux fonctions isocyanates, avantageusement plus de deux, (possibilité de valeurs fractionnaires puisqu'il s'agit en général de mélange d'oligomères plus ou moins condensés) lequel est lui-même le plus souvent issu d'une précondensation ou d'une prépolymérisation de diisocyanate unitaire.

D'une manière générale, les masses moléculaires moyennes de ces prépolymères ou de ces précondensats sont au plus égales à 2000 (un chiffre significatif), plus couramment à 1000 (un chiffre significatif de préférence deux).

Ainsi, parmi les polyisocyanates utilisés pour l'invention, on peut citer ceux de type biuret et ceux dont la réaction de di- ou trimérisation a conduit à des cycles à quatre, cinq ou six chaînons. Parmi les cycles à six chaînons, on peux citer les cycles isocyanurique issus d'une homo- ou d'une hétéro-trimérisation de divers diisocyanates seuls, avec d'autres isocyanate(s) [mono-, di--, ou poly-isocyanate(s)] ou avec du gaz carbonique (ou bioxyde de carbone). Dans ce cas, on remplace un azote du cycle isocyanurique par un oxygène.

Les polyisocyanates préférés sont ceux qui présentent au moins une fonction isocyanate aliphatique. En d'autres termes, au moins une fonction isocyanate masquée selon l'invention est reliée au squelette par l'intermédiaire d'un carbone de type SP3 portant avantageusement un atome d'hydrogène, de préférence deux.

Le dérivé aromatique hydroxylé sur le noyau, servant à masquer la fonction isocyanate est avantageusement choisi parmi ceux de formule (I) :

$$Ar(R)_n(Y\text{-}Z)_m(OH)p \qquad (I)$$

où Ar est un reste aromatique sur lequel est greffé n substituants R, m fonctions polaires Z choisies parmi les nitriles et les groupements carbonyles, et p fonctions hydroxyles.

Les valeurs de n, m et p sont telles que la somme n + m + p soit au plus égale au nombre de sommets substituables, avantageusement p est au plus égal à 2, de préférence p est égal à 1.

Avantageusement, m est au plus égal à deux, de préférence m est égal à 1.

Avantageusement n est au plus égal à 3, de préférence choisi parmi zéro, un, et deux, plus préférentiellement égal à zéro.

R représente des substituants indifférents pour la réaction de masquage et en général correspond à des chaînes hydrocarbonées, le plus souvent des chaînes alcoyles au sens étymologique du terme, à savoir un alcool auquel on a enlevé sa fonction hydroxyle.

Deux substituants vicinaux R peuvent être reliés entre eux pour former un cycle, qui peut être aromatique par exemple.

Z est avantageusement choisi parmi les groupements présentant une fonction carbonyle. Parmi ces fonctions, il convient de citer les fonctions alcoxycarbonyles (ou en d'autres termes les fonctions esters), la fonction amide, la fonction cétone avec la condition préférentielle qu'il n'y ait pas d'hydrogène acide (en d'autres termes la fonction n'est avantageusement pas porteuse d'hydrogène ou si elle en porte, le pKa correspondant est au moins égal à environ 20 (un chiffre significatif, de préférence deux) plus préférentiellement au moins égal à environ 25 en a de la fonction carbonyle (ester, cétone ou amide). Ainsi, les amides (y compris lactame, voire urée) préférés sont avantageusement substitués, de préférence suffisamment pour qu'il n'y ait pas d'hydrogène sur l'azote de la fonction amide ou de manière qu'il n'y ait pas d'hydrogène réactif.

où Y est choisi parmi les groupements divalents

avantageusement -O-, -S-, NR'-, -CR'R''- avec R' et R'' choisi parmi les radicaux hydrocarbonés, avantageusement alcoyle, de 1 à 6 atomes de carbone, avantageusement de 1 à 4, de préférence méthyle, plus préférentiellement hydrogène et de préférence Y représente une liaison simple.

Il est préférable que la ou les fonctions polaires Z (en général choisies parmi la fonction nitrile et/ou les fonctions carbonyles) ne soient pas vicinales du groupe hydroxyle comme par exemple dans l'acide salicylique.

Le reste aromatique Ar est constitué d'un ou plusieurs noyaux avantageusement condensés, hétéro- ou homocycliques.

Il est préférable que Ar ne comporte pas plus de deux noyaux, et de préférence pas plus d'un noyau.

Le reste aromatique Ar peut être constitué d'un ou plusieurs noyaux hétéro- ou homocycliques. Le plus souvent homocyclique en raison de leur facilité d'accès. Il convient toutefois de souligner l'intérêt des hétérocycles à 6 chaînons qui présente une température de libération très inférieure à celle des homocycles correspondants.

Il est souhaitable que le nombre total de carbones du dérivé aromatique hydroxylé sur le noyau soit au plus égal à 20, de préférence à 10 (un chiffre significatif).

Ce noyau est avantageusement à 6 chaînons, les chaînons étant constitués de carbone au d'azote avec le nombre de substituants nécessaires à la valence de ces atomes.

Parmi les acides donnant les résultats les plus satisfaisants, il convient de citer les acides greffés sur un noyau benzénique ou sur des noyaux pyridiniques. Ainsi, les acides metahydroxy- ou parahydroxybenzoïques et surtout leurs esters donnent de bons résultats.

Comme cela a déjà été mentionné, selon la présente invention, est préférable que le peint de fusion du composé ou du mélange de composés obtenu présente un point de fusion apparent au moins égal à 30°C, de préférence 50°C.

Il est également préférable que la température de transition vitreuse soit au moins égale à 20°C, avantageusement à 40°C

Il est préférable de choisir les composés selon la présente invention de manière à ce qu'ils réagissent complètement avec un alcool primaire à 250°C en moins d'une demi-heure.

On considère que la réaction est complète si elle est réalisé à 90% ou plus.

Les isocyanates pour lesquels l'invention est la plus intéressante sont ceux dont l'atome d'azote est lié à un carbone d'hybridation $sp^3$ et plus particulièrement aux isocyanates aliphatiques, et notamment aux polyméthylènes diisocyanates et leurs différents dérivés de condensation (biuret, etc......) de di- et de "trimérisation".

Selon la présente invention. Il est souhaitable et parfois nécessaire que le pourcentage de fonction isocyanate libre résiduelle soit au plus égal à 5%, avantageusement à 3 %, de préférence à 1 %. Les points de fusion ou de transition vitreuse les plus élevés sont obtenus avec des pourcentages ne dépassant pas 0,5 %. Les teneurs en dérivé aromatique hydroxylé sur le noyau sont également avantageusement faibles, c'est-à-dire au plus égales à 5 %, avantageusement à 3%, de préférence à 1%.

Comme cela a été mentionné au début de la présente description, la présente invention vise également des compositions de poudres qui comportent un polyisocyanate masqué ou un mélange de polyisocyanates masqués, selon la présente invention.

Dans la présente description, les caractéristiques de granulométrie font souvent référence à des notations du type $d_n$ où n est un nombre de 1 à 99, cette notation est bien connue dans de nombreux domaines techniques, mais est un peu plus rare en chimie. Aussi, peut-il être utile d'en rappeler la signification. Cette notation représente la taille de particule telle que n % (en poids, ou plus exactement en masse, puisque le poids n'est pas une quantité de matière mais une force) des particules soit inférieur ou égal à la dite taille.

Dans les compositions de poudre selon cette mise en oeuvre, les isocyanates masqués selon la présente invention, constituent avantageusement une population (avantageusement distincte de celle des coréactifs) de particules dont le $d_{90}$ est au plus égal à 200 microns, avantageusement à 100 microns, de préférence à 50 microns. Cette population de particules présente un $d_{10}$ au moins égal à 1 micron, avantageusement à 5 microns de préférence à 10 microns.

Les compositions des poudres comportent avantageusement au moins un polyol (au moins diol) ou, dans certains cas, des polyamines. On peut également avoir des composés polyfonctionnels présentant au moins deux fonctions choisies parmi les fonctions amines ou -ols (phénols ou de préférence alcools). Les composés ci-dessus peuvent présenter en outre d'autre fonctions (par exemple fonction acide telle que carboxylique ou sulfonique) à condition que ces fonctions n'empêchent pas la condensation ou la réticulation.

Ces polyols ou polyamines forment également eux-mêmes des poudres et répondent aux mêmes contraintes de point de fusion et de température de transition vitreuse que celles évoquées précédemment.

Il est préférable que le point de fusion des compositions selon la présente invention présente un point de fusion au moins égal à 50°C. Il est même souhaitable que les températures de ramollissement soit telles qu'il n'y ait pas frittage de la poudre à une température d'au moins 50°C.

Il est également préférable que leur température de transition vitreuse soit au moins égale à 40°C.

Avantageusement, les compositions de poudres comportent également au moins un catalyseur, en général et de préférence des catalyseurs à base d'étain ou de zinc.

Le cas échéant, elles comportent des additifs usuels en la matière, tels que des charges, des pigments ($TiO_2$... ) des additifs d'amélioration des propriétés physiques (tension superficielle, résistance au vieillissement, à la lumière, facilité de mise en oeuvre...) Selon la présente invention, un procédé de synthèse consiste à mettre en contact dans un solvant l'isocyanate libre, ou partiellement libre, avec le composé hydroxyaromatique, c'est-à-dire le composé de type phénolique. Lorsque les composés selon l'invention et leur précurseurs sont stables dans les conditions ci-après, on peut réaliser la synthèse sans solvant mais à l'état fondu. Le produit final est alors refroidi, par exemple en fabriquant des écailles, qui peuvent être obtenues par le refroidissement brutal occasionné en versant le mélange réactionnel sur une paroi froide. Les écailles obtenues peuvent être broyées.

Il est important pour obtenir de bons (c'est-à-dire de faibles) pourcentages de fonction isocyanate libre résiduelle, d'introduire le dérivé aromatique hydroxylé sur le noyau en quantité très proche de la stoechiométrie. Il est préférable d'être en excès stoechiométrique léger (de 0,5 à 2%, de préférence au plus 1 %).

Il est également préférable d'ajouter un catalyseur de condensation des isocyanates sur des fonctions -ol. Ces catalyseur de condensation sont souvent à base d'étain ou d'amine tertiaire.

La température de fin de condensation est avantageusement au plus égale à 100°C (un chiffre significatif de préférence deux) de préférence à 80°C et avantageusement au moins égale 50°C, de préférence à 60°C. En effet si l'on chauffe trop, le pourcentage d'isocyanate libre risque d'être trop élevé.

Lorsqu'il y a solvant, il est de préférence choisi de manière à être suffisamment polaire pour dissoudre au moins 50, de préférence au moins 100, préférentiellement au moins 200 grammes par litre de l'isocyanate initial.

Une fois la réaction terminée, il convient de précipiter le produit final, soit selon une technique classique de cristallisation, et plus préférentiellement par addition d'un composé précipitant suffisamment peu polaire pour provoquer la précipitation sans qu'il y ait nécessairement cristallisation.

Le composé précipitant est bien évidemment un composé de type volatil, et le plus souvent des composés du type mélange d'hydrocarbures légers, du type éther de pétrole ou du type hexane ou heptane. On peut également utiliser, seuls ou en mélange, les éthers d'alcool léger (c'est à dire les alcools comportant au plus six atomes de carbones de préférence 4).

Généralement, on utilise des composés du type alcane ou alcène dont le nombre de carbones est inférieur à 20 et supérieur à 4.

Les exemples non limitatifs suivants illustrent l'invention.

### *Exemple 1*

**Synthèse du Tolonate HDT bloqué p-Hydroxy benzoate de méthyle :**

Dans un réacteur de 500 ml, charger :

- trimère de l'hexaméthylène diisocyanate vendu sous la marque Tolonate HDT® = 54,2 g (indice NCO - 22,1 %)

- Solvesso 100®- 25g.

Sous agitation et à température ambiante, ajouter en plusieurs fois.

- p-Hydroxy benzoate de méthyle = 47,6 g (0,31 mole).

La masse réactionnelle est chauffée jusqu'à 60°C et maintenue à cette température jusqu'à disparition des fonctions NCO.

Après refroidissement, le produit désiré (polyisocyanate bloqué) précipite. Il est réduit en poudre et lavé au moyen de n-hexane.

- n Hexane = 41,2 g.

La masse réactionnelle est filtrée, le solide obtenu lavé par plusieurs tractions d'hexane puis broyé et séché à nouveau.

- masse obtenue = 95,7 g

- point de fusion = 85°C.
  RMN on remarque 3 pics à

  7,8. ppm (hydrogène porté par l'azote du carbamate)
  7,9. ppm (hydrogène aromatique en ortho de la fonction ester carbamique)
  7,10 ppm (hydrogène aromatique en ortho de la fonction carbonyle)

EP 0 943 638 B1

### *Exemple 2*

**Formulation vernis du produit précédent :**

Le produit intermédiaire obtenu (I) est formulé de la façon suivante :

- I = 6,0 g
- Desmophen 690® = 14,0 g (% OH = 2 %)

Soit un rapport NCO/OH = 1

Le mélange des deux poudres est broyé jusqu'à obtenir un mélange parfaitement homogène d'une granulométrie inférieure à 50 $\mu$m.

Une fraction de cette poudre est appliquée en couche d'épaisseur 300 $\mu$m sur plaque acier et traitée thermiquement à différentes températures pendant 30 ou 60 minutes.

| CUISSON | 30 minutes | | 60 minutes | |
|---------|------------|---|------------|---|
| | Test Solvant (2) | Dureté à chaud (1) | Test solvant (2) | Dureté à chaud (1) |
| 130°C | D | M | D | M |
| 160°C | D | M | D | M |
| 190°C | D | M | I | TB |
| 200°C | I | TB | I | TB |

Le film obtenu est qualifié par sa dureté et sa résistance au solvant :

(1) TB = Très bonne : M = Médiocre

(2) Dépôt d'une goutte de Méthyl - Ethyl Cétone et observation de la détérioration du film,

D = le film est dégradé par l'action du solvant
I = le film est intact après action du solvant

Mode Opératoire :

**Exemple 3** (exemple comparatif)

Introduire dans un réacteur les produits suivants :

- Tolonate HDT ® : 100 grs (0.529 mole NCO)

Ajouter

- p Hydroxy benzoate de Méthyle : 81.3 grs (0.529 mole)

Chauffer sous agitation jusqu'à obtenir la fusion de l'agent bloquant vers 85 °C , vers 100 °C le milieu est totalement limpide et incolore.

Chauffer à 120 °C et maintenir 1 h à cette température.

Après refroidissement, le produit se présente sous la forme d'une gomme dure légèrement collante

$$Tg = 8 \ °C$$

on détermine par dosage à la dibutylamine la teneur en fonction NCO libre est d'environ 10 %

**Exemple _4_**

Introduire dans un réacteur les produits suivants :

-   Tolonate HDT ® : 100 grs (0.529 mole NCO)

Ajouter

-   p Hydroxy benzoate de Méthyle : 81.3 grs (0.529 mole)
-   Triéthyl amine (TEA) : 0.2 grs

Chauffer sous agitation jusqu'à obtenir la fusion de l'agent bloquant vers 85 °C , vers 100 °C le milieu est totalement limpide et incolore.
Après maintien à 100 °C, on mesure par dosage à la dibutyl-amine la teneur en fonctions NCO libres.

|  |  |  |
|---|---|---|
| 1 h à 100 °C : | NCO libres= | 6.2 % |
| 2h à 100°C: | NCO | 5.6% |
| 5hà100°C: | NCO | 5.6% |

**Exemple _5_**

Introduire dans un réacteur les produits suivants :

-   Tolonate HDT ® : 100 grs (0.529 mole NCO)

Ajouter

-   p Hydroxy benzoate de Méthyle : 81.3 grs (0.529 mole)
-   Triéthyl amine (TEA) : 0.2 grs

Chauffer sous agitation jusqu'à obtenir la fusion de l'agent bloquant vers 85 °C , vers 100 °C le milieu est totalement limpide et incolore.
La masse réactionnelle est ensuite refroidie progressivement à 60 °C puis maintenue à cette température .
On mesure par dosage à la dibutyl-amine la teneur en fonctions NCO libres.

|  |  |  |
|---|---|---|
| 30 min à 60 °C : | NCO libres= | 3.1 % |
| 1 h à 60°C : | NCO | 2.6% |
| 4 h à 60°C : | NCO | 2.6% |

**Exemple _6_**

Introduire dans un réacteur les produits suivants :

-   Tolonate HDT ® : 100 grs (0.529 mole NCO)

Ajouter

-   p Hydroxy benzoate de Méthyle : 81.3 grs (0.529 mole)
-   Triéthyl amine (TEA) : 0.5 grs

Chauffer sous agitation jusqu'à obtenir la fusion de l'agent bloquant vers 85 °C vers 100 °C le milieu est totalement limpide et incolore.
La masse réactionnelle est ensuite refroidie progressivement à 60 °C puis maintenue à cette température.
On mesure par dosage à la dibutyl-amine la teneur en fonctions NCO libres.

|  |  |  |
|---|---|---|
| 3 h à 60 °C : | NCO libres= | 1.2 % |

On rajoute dans le milieu réactionnel à nouveau 0.5 g de TEA puis après un nouveau maintien, on dose les fonctions NCO libres :

$$3 \text{ h à } 60°C: \quad NCO \quad 0.2\%$$

Après refroidissement, le produit se présente sous la forme d'une gomme dure qui peut être broyée.

$$Tg = 24 \text{ °C}$$

**Exemple _7_** (exemple comparatif)

Mode opératoire identique à l'exemple 4 mais en utilisant à la place du p Hydroxybenzoate de méthyle, le produit suivant :

p-Hydroxybenzoate d'Ethyle : 88.9 g (0.529 mole)
Après refroidissement, le produit se présente sous la forme d'une gomme solide

$$Tg = 18.8 \text{ °C}$$

**Exemple _8_** (exemple comparatif)

Mode opératoire identique à l'exemple 4 mais en utilisant à la place du p Hydroxybenzoate de méthyle, le produit suivant :

p-Hydroxybenzoate de Butyle : 77.8 g (0.529 mole)

Après refroidissement, le produit se présente sous la forme d'une liquide vitreux.

$$Tg = 5.5 \text{ °C}$$

**Exemple _9_**

Mode opératoire identique à l'exemple 4 mais en utilisant à la place du p Hydroxybenzoate de méthyle, le produit suivant :

p-Hydroxybenzoate d'Isopropyle : 73.1 g (0.529 mole)

Après refroidissement, le produit se présente sous la forme d'une gomme solide.

$$Tg = 23 \text{ °C}$$

**_Exemple 10_**

**Formulation vernis du produit précédent :**

Le produit intermédiaire obtenu (I) Tolonate HDT bloqué p-Hydroxy benzoate de Méthyle est formulé de la façon suivante :

- I = 38.3 g
- Johnson 587 [®] = 61.7 g (% OH = 2.8 %)
  Soit un rapport NCO/OH = 1.1

Le mélange des deux poudres est broyé jusqu'à obtenir un mélange parfaitement homogène d'une granulométrie inférieure à 50 μm.

Une fraction de cette poudre est appliquée en couche d'épaisseur 200 μm sur plaque acier et traitée thermiquement à différentes températures pendant 30 minutes.

| CUISSON | 30 minutes | |
|---|---|---|
| | Test Solvant (2) | Dureté à chaud (1) |
| 130°C | D | M |
| 140°C | D | M |
| 150°C | D | M |
| 160°C | I | TB |

Le film obtenu est qualifié par sa dureté et sa résistance au solvant :

(1) TB = Très bonne : M = Médiocre

(2) Dépôt d'une goutte de Méthyl - Ethyl Cétone et observation de la détérioration du film,

D =      le film est dégradé par l'action du solvant

=      le film est intact après action du solvant

**Revendications**

1. Polyisocyanate masqué, **caractérisé par** le fait qui peut être obtenu par la condensation avec un isocyanate d'un dérivé aromatique hydroxylé sur le noyau et portant un groupe présentant une fonction choisie parmi les fonctions acide carboxylique et nitrile et dont le point de fusion espace ou le point de fusion apparent dans le cas des composés n'ayant pas un point de fusion franc, mesuré au banc koffler ou à l'aide d'une méthode de type capillaire est au moins égal à 30°C et le point de transition vitreuse au moins égal à 10°C.

2. Polyisocyanate masqué selon la revendication 1, **caractérisé par le fait qu'**il comporte au moins une fonction isocyanate masquée selon invention reliée au squelette par d'intermédiaire d'un carbone de type sp$^3$ portant un atome d'hydrogène, de préférence deux.

3. Polyisocyanate masqué selon l'une des revendications 1 et 2, **caractérisé par le fait que** ledit diisocyanate élémentaire est un polyméthylène diisocyanate.

4. Polyisocyanate masqué selon l'une des revendications 1 à 3, **caractérisé par le fait que** ledit polyisocyanate est un dérivé de condensation et/ou de di- et trimérisation d'un ou plusieurs diisocyanates élémentaires aliphatiques.

5. Polyisocyanate masqué selon l'une des revendications 1 à 4, **caractérisé par le fait que** ledit polyisocyanate est un composé de type biuret ou trimère d'un ou plusieurs diisocyanates.

6. Polyisocyanate masqué selon l'une des revendications 1 à 5, **caractérisé par le fait que** ledit polyisocyanate est un dérivé de trimérisation d'un polyméthylène diisocyanate.

7. Polyisocyanate masqué selon l'une des revendications 1 à 6, **caractérisé par le fait que** ledit diisocyanate élémentaire aliphatique est l'héxaméthylène diisocyanate.

8. Polyisocyanate masqué selon l'une des revendications 1 à 7, **caractérisé par** de rait que ledit dérivé aromatique hydroxylé répond à la formule (I)

$$Ar(R)_n(Y\text{-}Z)_m(OH)_P \qquad (I)$$

- où Ar est un reste aromatique;

- où R représente un substituant hydrocarboné, en général alcoyle:
- où Z est choisi parmi les fonctions nitrile et acide carbonique :
- où Y est choisi parmi les groupements divalents, avantageusement une liaison simple,
- O-, -S- NR'-.-CR'R''- avec R' et R'' choisis parmi les radicaux hydrocarbonés, avantageusement alcoyle, de 1 à 6 atomes da carbone, avantageusement de 1 à 4. de préférence méthyle, plus préférentiellement hydrogène ;
- et où n+m+p est au plus égal au nombre de sommets substituables de Ar, avec n choisi parmi 0, 1, 2, et 3, m est choisi parmi 1 et 2 et p est choisi parmi 1 et 2.

9. Polyisocyanate masqué selon la revendication 8, **caractérisé par le fait que** p est égal à 1 et que m est avantageusement au plus égal à 2.

10. Polyisocyanate masqué selon l'une des revendications 8 ou 9, **caractérisé par le fait que** la fonction hydroxyle n'est pas vicinale d'une ou de la fonction Z.

11. Polyisocyanate masqué selon l'une des revendications 8 à 10, **caractérisé par le fait que** Ar est choisi parmi les noyaux aromatiques à six chaînons carbonés ou azotés.

12. Polyisocyanate masqué selon l'une des revendications 1 à 11, **caractérisé par le fait que** ledit dérivé aromatique hydroxylé est l'acide para- ou métahydroxybenzoïque.

13. Polyisocyanate masqué selon la revendication 11, **caractérisé par le fait que** ledit dérivé hydroxylé aromatique est le parahydroxybenzonitrile.

14. Polyisocyanate masqué selon l'une des revendications 1 à 15, **caractérisé par le fait que** son point de fusion ou son point de fusion apparent dans le cas des composés n'ayant pas un point de fusion franc, mesuré au banc koffler ou à l'aide d'une méthode de type capillaire est au moins égal à 50°C.

15. Composition de poudres, notamment pour revêtement, **caractérisée par le fait qu'**elle comporte au moins un des polyisocyanates masqués, selon l'une des revendications 1 à 14, ledit polyisocyanate masqué étant sous la forme d'une poudre.

16. Composition selon la revendication 15, **caractérisée par le fait qu'**elle comporte en outre un catalyseur à base de zinc ou d'étain.

17. Composition selon les revendications 15 et 16, **caractérisée par le fait qu'**elle contient en outre une poudre de polyol.

18. Composition selon les revendications 15 à 17, **caractérisée par** la fait qu'elle contient en outre une polyamine sous forme de poudre.

19. Procédé de synthèse d'un isocyanate masqué selon les revendications 1 à 14, **caractérisé par** de fait qu'il comporte la mise en contact dudit dérivé aromatique hydroxylé avec ledit isocyanate à une température au plus égale à 100°C, en fin de condensation.

20. Procédé selon la revendication 19, **caractérisé par le fait qu'**il comporte en outre l'étape de précipitation de l'isocyanate masqué au moyen de l'addition d'un solvant apolaire de type alcane ou alcène.

21. Procédé selon la revendication 20, **caractérisé par le fait que** le solvant apolaire de l'étape de précipitation est un alcane de $C_4$ à $C_{20}$.

22. Procédé selon l'une des revendications 20 ou 21, **caractérisé par le fait que** ledit isocyanate libre est placé dans un solvant, ledit solvant est choisi de manière à ce que l'isocyanate soit soluble à hauteur d'au moins 50 grammes, avantageusement 100 grammes, de préférence 200 grammes par litre.

**Claims**

1. Masked polyisocyanate **characterised by** the fact that it may be obtained by the condensation with an isocyanate of a aromatic derivative which is hydroxylated at the nucleus and which carries a group which offers a function selected from carboxylic acid and nitrile functions, and whose melting point or apparent melting point in the case of compounds which have no clear melting point, measured using a Koffler bench or a capillary type method, is equal to at least 30°C and whose glass transition point is equal to at least 10°C.

2. Masked polyisocyanate according to claim 1 **characterised by** the fact that it includes at least one masked isocyanate function according to the invention connected to the skeleton through an sp3 type carbon bearing an atom of hydrogen and preferably two.

3. Masked polyisocyanate according to one of claims 1 and 2, **characterised by** the fact that the said elementary diisocyanate is a polymethylene diisocyanate.

4. Masked polyisocyanate according to one of claims 1 to 3, **characterised by** the fact that the said polyisocyanate is a derivative of condensation and/or of di- and trimerisation of one or more elementary aliphatic diisocyanates.

5. Masked polyisocyanate according to one of claims 1 to 4, **characterised by** the fact that the said polyisocyanate is a biuret-type compound or a trimer of one or more aliphatic diisocyanates.

6. Masked polyisocyanate according to one of claims 1 to 5, **characterised by** the fact that the said polyisocyanate is a trimerisation derivative of a polymethylene diisocyanate.

7. Masked polyisocyanate according to one of claims 1 to 6, **characterised by** the fact that the said elementary aliphatic diisocyanate is hexamethylene diisocyanate.

8. Masked polyisocyanate according to one of claims 1 to 7, **characterised by** the fact that the said hydroxylated aromatic derivative is in accordance with formula (i).

$$Ar(R)_n(Y\text{-}Z)_m(OH)_p \qquad (i)$$

- where Ar is an aromatic residue;
- where R represents a hydrocarbon containing substituant, generally alkoyl;
- where Z is selected from nitrile and carboxylic acid functions;
- where Y is selected from divalent groups, advantageously a single bond,
- O-, -S-, NR'-, -CR'R''- where R' and R'' are selected from hydrocarbon containing radicals, advantageously alkoyl, with 1 to 6 carbon atoms, advantageously with 1 to 4, preferably methyl and more preferably hydrogen.
- and where $n + m + p$ is at most equal to the number of the points on Ar which can be substituted, where n is selected from amongst 0, 1, 2 and 3, m is selected from 1 and 2 and p selected from 1 and 2.

9. Masked polyisocyanate according to claim 8, **characterised by** the fact that p is equal to 1 and that m is advantageously at most equal to 2.

10. Masked polyisocyanate according to one of claims 8 or 9, **characterised by** the fact that the hydroxyl function is not vicinal to one or to the function Z.

11. Masked polyisocyanate according to one of claims 8 to 10, **characterised by** the fact that Ar is selected from aromatic nuclei with six carbon-containing or nitrogen-containing links.

12. Masked polyisocyanate according to one of claims 1 to 11, **characterised by** the fact that the said hydroxylated aromatic derivative is para- or meta- hydroxybenzoic acid.

13. Masked polyisocyanate according to claim 11, **characterised by** the fact that the said aromatic hydroxylated derivative is parahydroxybenzonitrile.

14. Masked polyisocyanate according to one of claims 1 to 15, **characterised by** the fact that its melting point or apparent melting point in the case of compounds which do not have a clear melting point, measured on a Koffler bench or using a capillary type method, is equal to at least 50°C.

15. Composition of powders, in particular for coating, **characterised by** the fact that it contains at least one of the masked polyisocyanates, according to one of claims 1 to 14, where the said masked polyisocyanate is in the form of a powder.

16. Composition according to claim 15, **characterised by** the fact that it includes in addition a zinc- or tin-based catalyst.

17. Composition according to claims 15 and 16, **characterised by** the fact that it contains in addition a polyol powder

18. Composition according to claims 15 to 17, **characterised by** the fact that it contains in addition a polyamine in the form of a powder.

19. Process for the synthesis of a masked isocyanate according to claims 1 to 14, **characterised by** the fact that it involves bringing the said hydroxylated aromatic derivative into contact with the said isocyanate at a temperature which is at most equal to 100°C, at the end of condensation.

20. Process according to claim 19, **characterised by** the fact that it includes in addition a step for precipitation of the masked isocyanate by means of addition of an alkane- or alkene-type apolar solvent,

21. Process according to claim 20, **characterised by** the fact that the apolar solvent for the precipitation step is a $C_4$ to $C_{20}$ alkane.

22. Process according to one of claims 20 or 21, **characterised by** the fact that the said free isocyanate is placed in a solvent, the said solvent is selected so that the isocyanate is soluble up to 50 grams, advantageously 100 grams and preferably 200 grams per litre.

**Patentansprüche**

1. Maskiertes Polyisocyanat, **dadurch gekennzeichnet, dass** es durch Kondensation eines am Ring hydroxylierten aromatischen Derivates, das eine Gruppe trägt, die eine Funktion aufweist, die unter den Funktionen Karbonsäure und Nitril ausgewählt wurde, mit einem Isocyanat dargestellt werden kann und dessen Schmelzpunkt oder scheinbarer Schmelzpunkt im Fall der Verbindungen, die keinen wahren Schmelzpunkt aufweisen, gemessen auf der Koflerbank oder mit Hilfe einer Methode vom Kapillartyp, mindestens gleich 30°C ist und die Glasübergangstemperatur mindestens gleich 10°C.

2. Maskiertes Polyisocyanat nach Patentanspruch 1, **dadurch gekennzeichnet, dass** es mindestens eine erfindungsgemäß maskierte Isocyanatgruppe aufweist, die über ein Kohlenstoffatom vom Typ sp$^3$, das ein oder vorzugsweise zwei Wasserstoffatome trägt, an das Skelett gebunden ist.

3. Maskiertes Polyisocyanat nach einem der Patentansprüche 1 und 2, **dadurch gekennzeichnet, dass** das genannte Ausgangs-Diisocyanat ein Polymethylendiisocyanat ist.

4. Maskiertes Polyisocyanat nach einem der Patentansprüche 1 bis 3. **dadurch gekennzeichnet, dass** das genannte Polyisocyanat ein Derivat durch Kondensation und/oder Di- und Trimerisation eines oder mehrerer aliphatischer Ausgangs-Diisocyanate ist.

5. Maskiertes Polyisocyanat nach einem der Patentansprüche 1 bis 4, **dadurch gekennzeichnet, dass** das genannte Polyisocyanat eine Verbindung vom Typ Biuret oder Trimer eines oder mehrerer aliphatischer Diisocyanate ist.

6. Maskiertes Polyisocyanat nach einem der Patentansprüche 1 bis 5, **dadurch gekennzeichnet, dass** das genannte Polyisocyanat ein Trimerisationsderivat eines Polymethylendiisocyanats ist.

7. Maskiertes Polyisocyanat nach einem der Patentansprüche 1 bis 6, **dadurch gekennzeichnet, dass** das genannte aliphatische Ausgangs-Diisocyanat Hexamethylendiisocyanat ist.

8. Maskiertes Polyisocyanat nach einem der Patentansprüche 1 bis 7, **dadurch gekennzeichnet, dass** das genannte hydroxylierte aromatische Derivat der Formel (I) entspricht

$$Ar(R)_n(Y\text{-}Z)_m(OH)_p \qquad (I)$$

- worin Ar ein aromatischer Rest ist,
- worin R für einen Kohlenwasserstoff-Substituenten steht, im Allgemeinen für einen Alkylrest,
- worin Z unter den Nitril- und Karbonsäure-Gruppen gewählt wurde,
- worin Y unter den zweiwertigen Gruppen, vorzugsweise mit Einfachbindung, -O-, -S-, NR', -CR'R'' gewählt wurde, wobei R' und R'' unter den Kohlenwasserstoffradikalen, vorzugsweise den Alkylresten, mit 1 bis 6, vorzugsweise 1 bis 4, Kohlenstoffatomen, vorzugsweise Methyl, noch stärker bevorzugt Wasserstoff, gewählt wurde
- und worin n+m+p höchstens gleich der Anzahl substituierbarer Ecken von Ar ist, wobei n zwischen 0, 1, 2 und 3 gewählt wurde, m unter 1 und 2 und p unter 1 und 2.

9. Maskiertes Polyisocyanat nach Patentanspruch 8, **dadurch gekennzeichnet, dass** p gleich 1 ist und m vorteilhafterweise höchstens gleich 2.

10. Maskiertes Polyisocyanat nach einem der Patentansprüche 8 oder 9, **dadurch gekennzeichnet, dass** die Hydroxylgruppe nicht einer der oder der Gruppe(n) Z vizinal ist.

11. Maskiertes Polyisocyanat nach einem der Patentansprüche 8 bis 10, **dadurch gekennzeichnet, dass** Ar unter den aromatischen Ringen aus sechs Kohlenstoff- oder Stickstoffatomen gewählt wurde.

12. Maskiertes Polyisocyanat nach einem der Patentansprüche 1 bis 11, **dadurch gekennzeichnet, dass** das genannte hydroxylierte aromatische Derivat die Para- oder Metahydroxybenzoesäure ist.

13. Maskiertes Polyisocyanat nach Patentanspruch 11, **dadurch gekennzeichnet, dass** das genannte hydroxylierte aromatische Derivat Parahydroxybenzonitril ist.

14. Maskiertes Polyisocyanat nach einem der Patentansprüche 1 bis 15, **dadurch gekennzeichnet, dass** sein Schmelzpunkt oder sein scheinbarer Schmelzpunkt im Fall der Verbindungen, die keinen wahren Schmelzpunkt aufweisen, gemessen auf der Koflerbank oder mit Hilfe einer Methode vom Kapillartyp, mindestens gleich 50°C ist.

15. Zusammensetzung von Pulvern, insbesondere für Beschichtungen, **dadurch gekennzeichnet, dass** sie mindestens eines der maskierten Polyisocyanate nach einem der Patentansprüche 1 bis 14 enthält, wobei das genannte maskierte Polyisocyanat in Pulverform vorliegt.

16. Zusammensetzung nach Patentanspruch 15, **dadurch gekennzeichnet, dass** sie außerdem einen Katalysator auf Zink- oder Zinnbasis enthält.

17. Zusammensetzung nach den Patentansprüchen 15 und 16, **dadurch gekennzeichnet, dass** sie außerdem ein Polyol-Pulver enthält.

18. Zusammensetzung nach den Patentansprüchen 15 bis 17, **dadurch gekennzeichnet, dass** sie außerdem ein pulverförmiges Polyamin enthält.

19. Verfahren zur Synthese eines maskierten Isocyanats nach den Patentansprüchen 1 bis 14, **dadurch gekennzeichnet, dass** es den Schritt umfasst, das genannte hydroxylierte aromatische Derivat mit dem genannten Isocyanat bei einer Temperatur in Berührung zu bringen, die am Ende der Kondensation höchstens gleich 100°C ist.

20. Verfahren nach Patentanspruch 19, **dadurch gekennzeichnet, dass** es außerdem den Schritt der Ausfällung des maskierten Isocyanats mit Hilfe des Zusatzes eines apolaren Lösungsmittels vom Typ Alkan oder Alken aufweist.

21. Verfahren nach Patentanspruch 20, **dadurch gekennzeichnet, dass** das apolare Lösungsmittel des Ausfällungsschrittes ein Alkan von $C_4$ bis $C_{20}$ ist.

22. Verfahren nach einem der Patentansprüche 20 oder 21, **dadurch gekennzeichnet, dass** das genannte freie Isocyanat in ein Lösungsmittel gebracht wird, wobei das genannte Lösungsmittel derart gewählt wird, dass das Isocyanat im Bereich von mindestens 50 Gramm, vorteilhafterweise 100 Gramm, vorzugsweise 200 Gramm pro Liter löslich ist.

**EP 0 943 638 B1**

## RÉFÉRENCES CITÉES DANS LA DESCRIPTION

**Documents brevets cités dans la description**

- FR 2266725
- EP 0562394 A